# Europäisches Patentamt

# European Patent Office

# Office européen des brevets

(11) Veröffentlichungsnummer: **0 161 340**
**B1**

(12) **EUROPÄISCHE PATENTSCHRIFT**

(45) Veröffentlichungstag der Patentschrift:
**24.06.87**

(21) Anmeldenummer: **84115380.2**

(22) Anmeldetag: **13.12.84**

(51) Int. Cl.⁴: **A 61 B 6/04,** A 61 B 5/05

㉞ **Patientenlagerungsvorrichtung.**

(30) Priorität: **04.05.84 DE 3416556**

(43) Veröffentlichungstag der Anmeldung:
**21.11.85 Patentblatt 85/47**

(45) Bekanntmachung des Hinweises auf die Patenterteilung:
**24.06.87 Patentblatt 87/26**

(84) Benannte Vertragsstaaten:
**DE FR GB**

(56) Entgegenhaltungen:
**EP - A - 0 132 785**
**DE - A - 2 400 403**
**FR - A - 1 317 341**
**US - A - 2 536 212**
**US - A - 2 567 566**
**US - A - 2 712 080**
**US - A - 3 541 334**
**US - A - 4 095 588**
**US - A - 4 244 021**
**US - A - 4 411 270**

(73) Patentinhaber: **Siemens Aktiengesellschaft Berlin und München, Wittelsbacherplatz 2, D-8000 München 2 (DE)**

(72) Erfinder: **Koob, Lothar, Sandstrasse 27a,**
**D-8551 Röttenbach (DE)**
Erfinder: **Riedl, Hermann, Dechsendorfer Strasse 6,**
**D-8520 Erlangen (DE)**
Erfinder: **Theil, Günter, Forellenweg 8, D-8520 Erlangen (DE)**
Erfinder: **Tschunt, Edgar, Im Winkel 9, D-8525 Rathsberg (DE)**

## Beschreibung

Die Erfindung betrifft eine Patientenlagerungsvorrichtung mit einem Sockel, auf dem eine Patientenlagerungsplatte längsverschiebbar gelagert ist, und mit mindestens einer am Patienten zu applizierenden Signalübertragungsvorrichtung, die über ein Kabel an einem ortsfesten Geräteteil angeschlossen ist.

Eine Patientenlagerungsvorrichtung dieser Art wird beispielsweise für die Anfertigung von Kernspintomogrammen benutzt und dient dabei zum Einführen eines Patienten in das Innere eines Magneten. Für die Auslenkung bestimmter Teilchen des Patienten, z. B. der Protonen, dient dabei eine Hochfrequenz-Sende- und -Empfangsspule, die nach dem Abschalten des Anregungsimpulses auch zur Bildung eines Signales dient, das durch das Zurückpendeln der angeregten Teilchen in ihre Ausgangslage gebildet wird. Diese Spule wird dabei beispielsweise zum Messen im Kopfbereich des Patienten über den Kopf geschoben und muss dann über ein flexibles Kabel an einem ortsfesten Geräteteil angeschlossen werden. Führt dieses flexible Kabel direkt zum ortsfesten Geräteteil, so kann es beim Einführen der Patientenlagerungsplatte in den Magneten an dessen Rand anstossen und beschädigt werden. Ferner wird das Bedienungspersonal in diesem Fall durch das Kabel behindert.

Der Erfindung liegt die Aufgabe zugrunde, eine Patientenlagerungsvorrichtung der eingangs genannten Art so auszubilden, dass das Kabel zwischen der Signalübertragungsvorrichtung und dem ortsfesten Geräteteil kurz gehalten werden kann und den Untersuchungsvorgang nicht stört.

Diese Aufgabe ist erfindungsgemäss dadurch gelöst, dass an der Patientenlagerungsplatte mindestens eine Steckvorrichtung für den Anschluss des Kabels der Signalübertragungsvorrichtung fest angeordnet ist, von der ein fest unter der Patientenlagerungsplatte verlegtes Kabel zum ortsfesten Geräteteil führt, wobei das Kabel eine Schleife zum Ermöglichen der Längsverschiebung der Patientenlagerungsplatte bildet. Bei der erfindungsgemässen Patientenlagerungsvorrichtung wird der ortsfeste Geräteteil zweckmässigerweise im Bereich des Sockels angeordnet, so dass das zwischen ihm und der Steckvorrichtung verlegte Kabel, das bei der Längsverschiebung der Patientenlagerungsplatte mitbewegt wird, nicht stört. Das Kabel zwischen der Signalübertragungsvorrichtung und der Steckvorrichtung kann kurz gehalten werden und führt nicht von der Patientenlagerungsvorrichtung weg, so dass auch dadurch keine Behinderungen vor und während der Untersuchung bedingt sind.

Die Erfindung ist nachfolgend anhand eines in der Zeichnung dargestellten Ausführungsbeispieles näher erläutert. Es zeigen:

Fig. 1 und 2 zwei schematische Darstellungen einer Patientenlagerungsvorrichtung in Verbindung mit einem Kernspintomographen zur Erläuterung des Erfindungsgedankens,

Fig. 3 eine Ansicht der Patientenlagerungsplatte der Patientenlagerungsvorrichtung gemäss den Figuren 1 und 2 von oben,

Fig. 4 eine Ansicht der Patientenlagerungsvorrichtung gemäss den Figuren 1 bis 3 von der Seite, und

Fig. 5 eine Einzelheit der Patientenlagerungsvorrichtung gemäss den Figuren 1 bis 4.

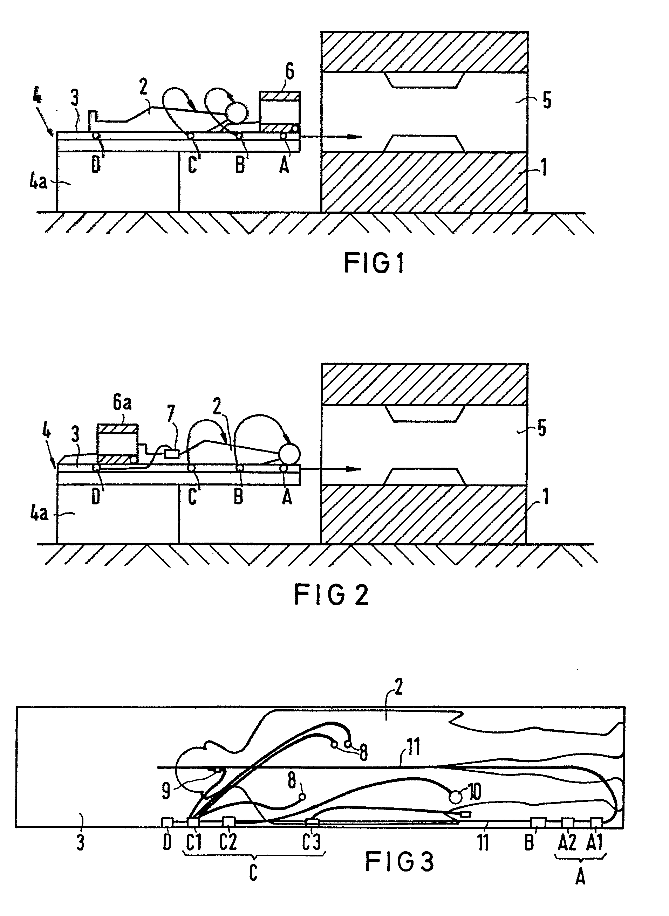

In den Figuren 1 und 2 ist der Magnet 1 eines Kernspintomographen dargestellt, der auf dem Fussboden des Behandlungsraumes befestigt ist. Die Untersuchung eines Patienten 2 erfolgt, indem dieser Patient mit Hilfe der Patientenlagerungsplatte 3 einer Patientenlagerungsvorrichtung 4 in den zylinderförmigen Raum 5 eingeschoben wird, der vom Magneten 1 gebildet wird. In der Figur 1 ist eine Spule 6 dargestellt, die zur Anregung der Wasserstoffkerne in einem vorbestimmten Bereich des Patienten 2, nämlich im Bereich des Kopfes, dient. Hierzu wird die Spule 6 auf den Kopf des Patienten 2 aufgeschoben und dieser dann auf der Patientenlagerungsplatte 3 in den Raum 5 eingeschoben. Durch den Magneten 1 werden die Wasserstoffkerne im Patienten 2 ausgerichtet und durch einen Hochfrequenzimpuls in der Spule 6 ausgelenkt. Die Spule 6 dient auch zum Erfassen des Rückpendelns der Wasserstoffkerne, so dass aus den gemessenen Signalen ein Bild der untersuchten Schicht des Patienten 2 berechnet werden kann.

Bei dem Beispiel gemäss Figur 2 ist eine Spule 6a vorgesehen, die um den Körper des Patienten 2 gelegt werden kann, d.h. zur Anregung und zum Messen im Bereich des Körperstammes dient. Ferner ist eine Sonderspule 7 vorhanden, die zum Anregen und Messen bestimmter Körperteile, z. B. des Knies, dient.

Aus den Figuren 1 und 2 geht hervor, dass die Patientenlagerungsplatte 3 Steckvorrichtungen A, B, C und D trägt, an die die Spulen 6, 6a, 7 sowie weitere Signalübertragungsvorrichtungen über Kabel angeschlossen werden können. Die Steckvorrichtung A dient dabei zum Anschluss der Spulen 6, 6a; die Steckvorrichtung B zum Anschluss eines Kopfhörers, die Steckvorrichtung C zum Anschluss von EKG-Elektroden, Atemmessvorrichtungen, Pulsmessvorrichtungen und dergleichen und die Steckvorrichtung D für den Anschluss der Sonderspule 7.

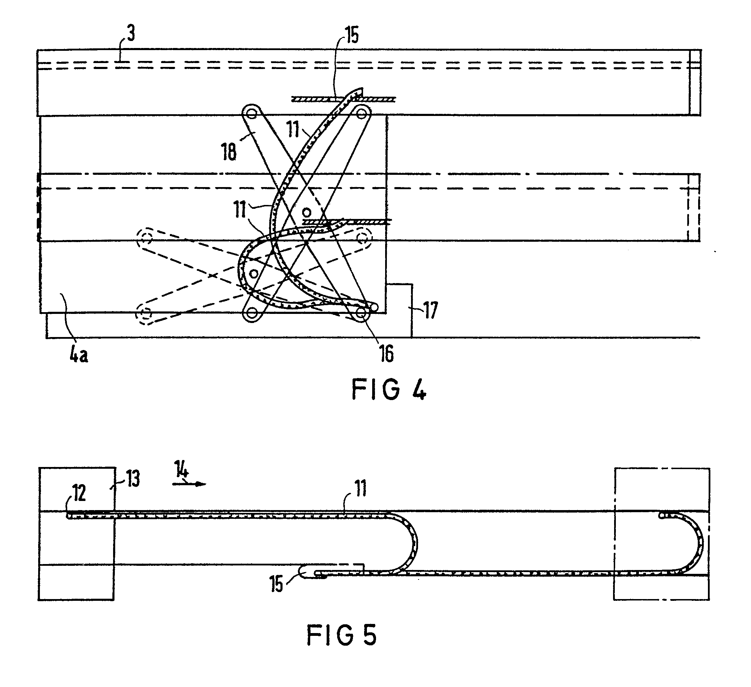

Die Figur 3 zeigt die Patientenlagerungsplatte 3 genauer. Auf dem Patienten 2 sind drei Elektroden 8 zur EKG-Abnahme aufgelegt, die zur Steckvorrichtung C1 geführt sind. Zur Steckvorrichtung C1 führt ferner die Leitung einer Atemsonde 9. Ein pneumatischer Handballenschalter 10 ist über eine Leitung an der Steckvorrichtung C2 angeschlossen und dient zur manuellen Auslösung der Steuervorgänge. Die Steckvorrichtung A ist ebenfalls aufgeteilt und zwar in zwei Steckvorrichtungen A1 und A2. Alle Steckvorrichtungen A bis D sind durch ein Kabel 11 miteinander verbunden, das unter der Patientenlagerungsplatte 3 fest verlegt ist.

Die Figur 5 zeigt, dass das Kabel 11 an einem Punkt 12 an einem Wagen 13 befestigt ist, der die Patientenlagerungsplatte 3 trägt, welche in der Figur 5 der Übersichtlichkeit halber nicht dargestellt ist. Der Wagen 13 ist aus der voll ausgezogen gezeichneten Stellung in die strichpunktiert gezeichnete Stellung verfahrbar. Das Kabel 11 bildet eine Schleife, die sich beim Verfahren des Wagens 13 in Richtung des Pfeiles 14 ebenfalls unter der Patientenlagerungsplatte 3 bewegt. Die Figur 5 zeigt dabei die Ansicht der Patientenlagerungsvorrichtung 4 von oben. Das Kabel 11 ist durch einen Durchbruch 15 geführt, der auch in der Seitenansicht gemäss Figur 4 sichtbar ist und führt über einen Punkt 16 zu einem ortsfesten Geräteteil 17. Es bildet zwischen den Punkten 15 und 16 ebenfalls eine Schleife, so dass die Patientenlagerungsplatte 3 mittels einer Hubvorrichtung 18 gehoben und gesenkt werden kann.

Die Teile 11 und 18 sind in der Figur 4 der Übersichtlichkeit halber voll gezeichnet, obwohl sie von der Verkleidung des Sockels 4a verdeckt werden.

## Patentanspruch

1. Patientenlagerungsvorrichtung mit einem Sockel (4a), auf dem eine Patientenlagerungsplatte (3) längsverschiebbar gelagert ist, und mit mindestens einer am Patienten (2) zu applizierenden Signalübertragungsvorrichtung (6, 6a, 7, 8, 9, 10), die über ein Kabel (11) an einem ortsfesten Geräteteil (17) angeschlossen ist, dadurch gekennzeichnet, dass an der Patientenlagerungsplatte (3) mindestens eine Steckvorrichtung (A bis D) für den Anschluss des Kabels der Signalübertragungsvorrichtung (6, 6a, 7, 8, 9, 10) fest angeordnet ist, von der ein fest unter der Patientenlagerungsplatte (3) verlegtes Kabel (11) zum ortsfesten Geräteteil (17) führt, wobei das Kabel (11) eine Schleife zum Ermöglichen der Längsverschiebung der Patientenlagerungsplatte (3) bildet.

## Claim

1. A support for a patient, comprising a base (4a) on which a patient support plate (3) is mounted so as to be longitudinally displaceable and comprising at least one signal transmitting device (6, 6a, 7, 8, 9, 10) which is to be applied to the patient (2) and which is connected via a cable (11) to a stationary device component (17), characterised in that the patient support plate (3) is permanently provided with at least one plug-in device (A-D) for the cable terminal of the signal transmitting device (6, 6a, 7, 8, 9, 10), from which plug-in device a cable (11), permanently installed under the patient support plate (3), leads to the stationary device component (17), where the cable (11) forms a loop which permits the longitudinal displacement of the patient support plate (3).

## Revendication

1. Dispositif de soutien pour patient comportant un socle (4a), sur lequel un plateau (3) formant couchette pour patient est monté de façon à être déplaçable longitudinalement, et comportant au moins un dispositif de transmission de signaux (6, 6a, 7, 8, 9, 10), devant être appliqué contre le patient (2) et qui est raccordé par l'intermédiaire d'un câble (11) à une partie fixe (17) de l'appareil, caractérisé par le fait que sur le plateau (3) formant couchette pour patient se trouve monté à demeure au moins un dispositif de connexion (A à D) prévu pour le raccordement du câble du dispositif de transmission de signaux (6, 6a, 7, 8, 9, 10) et dont un câble (11), fixé à demeure au-dessous du plateau (3) formant couchette pour patient, est raccordé à la partie fixe (17) de l'appareil, le câble (11) formant une boucle permettant le déplacement longitudinal du plateau (3) formant couchette pour patient.

FIG 1

FIG 2

FIG 3

FIG 4

FIG 5